Europäisches Patentamt

European Patent Office

Office européen des brevets

(11) Veröffentlichungsnummer : **0 079 398**
**B1**

(12) **EUROPÄISCHE PATENTSCHRIFT**

(45) Veröffentlichungstag der Patentschrift :
05.03.86

(21) Anmeldenummer : **81109677.5**

(22) Anmeldetag : **13.11.81**

(51) Int. Cl.⁴ : **D 04 H 1/42, A 61 L 17/00,
D 01 F 4/00**

(54) Verfahren zur Herstellung von Kollagenmaterial für chirurgische Zwecke.

(43) Veröffentlichungstag der Anmeldung :
**25.05.83 Patentblatt 83/21**

(45) Bekanntmachung des Hinweises auf die Patenterteilung : **05.03.86 Patentblatt 86/10**

(84) Benannte Vertragsstaaten :
**AT BE CH FR IT LI LU NL SE**

(56) Entgegenhaltungen :
**DE-B- 2 730 623**
**Die Akte enthält technische Angaben, die nach dem Eingang der Anmeldung eingereicht wurden und die nicht in dieser Patentschrift enthalten sind.**

(73) Patentinhaber : **Chemokol Gesellschaft zur Entwicklung von Kollagenprodukten
Spinnereistrasse 8
D-5190 Stolberg (DE)**

(72) Erfinder : **Steffan, Wolfgang
Schwaigfeldring 51
D-8425 Neustadt/Donau (DE)**

(74) Vertreter : **Hansen, Bernd, Dr.rer.nat. et al
Hoffmann, Eitle & Partner Patentanwälte Arabellastrasse 4
D-8000 München 81 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 079 398 B1

**Beschreibung**

Die Erfindung betrifft ein Verfahren zur Herstellung von Kollagenfasern für chirurgische Zwecke unter Verwendung von Achillessehnen oder Häuten als Ausgangsmaterial.

Die Herstellung von Kollagenfasern für chirurgische Zwecke, also als Nahtmaterial, oder zur Herstellung von Vliesen und dergleichen zum Blutstillen oder für Wundabdeckungen und ähnliche Anwendungen ist bekannt.

Bei der Verwendung von Kollagenmaterial bzw. Kollagenfasern für chirurgische Zwecke soll der native Charakter des Kollagens weitmöglichst erhalten bleiben. Dies bedeutet, daß man bei der Gewinnung von Kollagenfasern zwar das Ausgangsmaterial in geeigneter Weise aufschliessen und behandeln muß, daß das Endprodukt aber ein chemisch möglichst reines und sauberes Kollagen ist. Sofern es nicht aus medizinischen Zwecken erwünscht ist, daß die Kollagenfaser bestimmte Additive, z. B. Pharmaka, enthalten, soll das für chirurgische Zwecke verwendete Kollagenmaterial somit weitgehend frei von Fremdstoffen und Begleitstoffen sein.

Bekannt ist die Herstellung von chirurgischem Nahtmaterial aus rekonstituiertem und gegebenenfalls chromiertem Kollagen. Die vorliegende Erfindung befaßt sich nicht mit einem solchen rekonstituierten Kollagen, vielmehr betrifft sie ein Material, bei dem die ursprüngliche Faserstruktur des Kollagens im Endprodukt, wenn auch in modifizierter Form, erhalten bleibt.

Aus der DE-B-27 30 623 ist die Herstellung von Kollagenfasern bekannt. Bei dem bekannten Verfahren geht man von Haut und/oder Hautabfällen oder Sehnen aus, die alkalisch oder sauer aufgeschlossen werden. Dabei wird das zerkleinerte Ausgangsmaterial in einer ersten Stufe der Einwirkung starker Alkalien unterworfen und in einer sofort nachfolgenden Stufe in saurem Milieu gequollen. Das gequollene Material wird gewaschen, mechanisch aufgeschlossen, durch Zugabe geeigneter Salze oder organischer Lösungsmittel entquollen, gegebenenfalls fixiert und dann auf ca. 20 bis 40 Gew. % Feuchtigkeit, bezogen auf das Trockengewicht der Faser, entwässert.

Dann wird die Masse zu Fasern aufgelöst.

Zwar wird in der DE-B-27 30 623 erwähnt, daß die dort erhaltenen Kollagenfasern noppenfrei sind, jedoch hat man in der Praxis festgestellt, daß durch das unbefriedigende mechanische Auflösen des Kollagenmaterials die Fasern neben gutem fasrigen Material auch einen Anteil an Nichtfaserstoffen und Noppen (Faserknäuel, die nicht löslich sind) erhalten werden. Diese Noppen und Nichtfaserstoffe und der Anteil an kurzen Fasern verhindern die Weiterverarbeitung der so erhaltenen Kollagenfasern zu Nachfolgeprodukten, wie Fäden, Zwirne, Gewebe, Gewirke und Vliese, auf den üblichen in der Textilindustrie verwendeten Apparaturen.

Aufgabe der Erfindung ist es, ein Verfahren zur Herstellung von Kollagenfasern für chirurgische Zwecke zur Verfügung zu stellen, bei dem ein aufgeschlossenes, noppenfreies Kollagenmaterial hergestellt wird. Verbunden mit dieser Aufgabe ist es, das so hergestellte Kollagenmaterial in Faserform auf Textilverarbeitungsmaschinen, die entsprechend modifiziert und eingestellt werden, zu Garnen- und Flächengebilden zu verarbeiten.

Die Erfindung betrifft ein Verfahren zur Herstellung von Kollagenmaterial für chirurgische Zwecke, bei dem man Achillessehnen oder Häute alkalisch aufschließt und nach einer Säurebehandlung mechanisch behandelt, entquellt, vernetzt und trocknet, das dadurch gekennzeichnet ist, daß man die Achillessehnen oder Häute alkalisch bis zu einem Amidstickstoffgehalt von 0,15 bis 0,30 µMol/g aufschließt und nach einer Salzsäurebehandlung und Waschen auf einen pH-Wert von 2 bis 4 die angequollene Kollagenmasse unter Druck unter Ausbildung eines zweidimensionalen Netzwerkes in der Dicke reduziert, dann durch Hecheln einzelne längsorientierte Fasern freilegt und dann nach in an sich bekannter Weise erfolgter Entquellung die Fasern mit Hexamethylendiisocyanat in Gegenwart eines nichtionischen oder kationischen Emulgators vernetzt und trocknet und auf Textilmaschinen weiter verarbeitet.

Ausgangsmaterial bei dem erfindungsgemäßen Verfahren sind Achillessehnen oder Häute. Insbesondere Achillessehnen enthalten lange Kollagenfasern, die in nassem und trockenem Zustand in einer Richtung orientierbar sind, und die Herstellung von langen Fasern, die mechanisch weiter aufgespalten werden können, ermöglichen.

Achillessehnen und Häute sind als Abfall aus Schlachthöfen in großen Mengen preiswert erhältlich.

Die Achillessehnen oder Häute werden alkalisch bis zu einem Amidstickstoffgehalt von 0,15 bis 0,30 µMol/g, aufgeschlossen. Hierin unterscheidet sich schon der alkalische Aufschluß von dem alkalischen Aufschluß, der in der DE-B-27 30 623 gefordert wird. Nach den dortigen Angaben soll der alkalische Aufschluß bis zu einem Amidstickstoffgehalt von ca. 0,30 bis 0,40 Mol/g erfolgen. (Es wird angenommen, daß es richtig heißen müßte 0,30 bis 0,40 µMol/g, aber selbst dann ist die untere Grenze beim bekannten Verfahren die obere Grenze beim erfindungsgemäßen Verfahren.) Der Amidstickstoffgehalt von 0,15 bis 0,30 µMol/g ist sehr wesentlich für die nachfolgende Weiterverarbeitung des Kollagens.

Es ist ein Ausdruck für den Reifegrad des Kollagens. Damit wird der Kollagenzustand bezeichnet, in alle physikalisch aktiven Begleitstoffe entfernt werden können, wobei die Spaltung der Peptidbindungen und Quervernetzungen minimal bleiben. Die Alkalibehandlung wird in an sich bekannter Weise vorgenommen, z. B. mit Calciumhydroxid, wobei in diesem Falle die Alkalibehandlung mehrere Wochen bis zu etwa 6

Wochen dauern kann. Andere bekannte Verfahren, z. B. eine Behandlung mit Natriumhydroxid in Gegenwart von Natriumsulfat ermöglichen eine Abkürzung der Alkalibehandlung auf etwa 2 bis 5 Tage.

Während der Alkalibehandlung hat es sich als zweckmäßig erwiesen, daß man das zuvor behandelte Achillessehnen- oder Hautmaterial durch Riffelwalzenpaare führt, wodurch die Sehnen entwässert, geknickt und gepreßt werden. Man kann die Achillessehnen oder die Häute auch in Gegenwart von Alkali schlagen.

Nach der Alkalibehandlung bis zu einem Amidstickstoffgehalt von 0,15 bis 0,30 μMol/g wäscht man mit Salzsäure. Zwar ist ein Waschen mit anderen organischen oder auch anorganischen Säuren möglich, jedoch hat sich Salzsäure als zweckmäßig und vorteilhaft herausgestellt, insbesondere weil die dabei gebildeten Salze physiologisch unbedenklich sind und sich in einfacher Weise auch wieder herauswaschen lassen. Durch die Salzsäurebehandlung werden säureempfindliche Begleitstoffe, insbesondere Reste von Glykosaminoglykanen entfernt. Gleichzeitige erfolgt auch eine weitere Lockerung des Fasergefüges. Es ist zweckmäßig, daß man während der Salzsäurebehandlung mit Indikatorpapier kontrolliert, ob das Material in der ganzen Dicke gleichmäßig mit der Salzsäure in Berührung kommt. Der pH-Wert nach der Salzsäurebehandlung liegt unter 2.

Nach der Salzsäurebehandlung wird das Material auf einen pH von 2 bis 4, vorzugsweise 2, 5 bis 3, 5, durch Waschen mit fließendem Wasser gebracht.

Anschließend an die bisher geschilderte Verfahrensweise des alkalischen Aufschlusses, der Salzsäurebehandlung und dem Waschen, erfolgt eine mechanische Behandlung. Die nach dem Waschvorgang angequollene Kollagenmasse wird mechanisch z. B. mit Hilfe von Kalandern, Hammerpressen oder Riffelwalzen, behandelt. Bei diesem Vorgang werden die Fasern von dem klebenden löslichen Kollagen und von denaturiertem Kollagen befreit und auch teilweise entwässert. Durch diese mechanische Behandlung erfolgt eine Reduktion des dreidimensionalen Netzwerkes in der Dicke zu einem zweidimensionalen Netzwerk (der Ausdruck « zweidimensionales Netzwerk » ist hier so zu verstehen, daß die ursprünglich dreidimensional vernetzte Kollagenfasermasse in ihrer Dicke so reduziert wird, daß ein flaches, im wesentlichen zweidimensional vernetztes Gebilde entsteht, wobei auch ein derartig reduziertes flaches Gebilde selbstverständlich noch eine gewisse Höhe hat). Anschließend an die Dickenreduktion unter Druck unter Ausbildung des zweidimensionalen Netzwerkes wird die Kollagenfasermasse durch Hecheln in einzelne längsorientierte Fasern zerlegt. Dies kann auf einer üblichen Hechelmaschine erfolgen. Der Hechelvorgang kann/mehrfach, z. B. zwei- bis fünfmal wiederholt werden, wobei man die Kämmintensität bei den Wiederholungsstufen zweckmäßigerweise jeweils erhöht.

Die weitere Verarbeitung der in der vorstehend beschriebenen Weise durch Hecheln freigelegten einzelnen längsorientierten Fasern erfolgt in an sich bekannter Weise. Zunächst werden die einzelnen längsorientierten Fasern entquollen. Hierzu werden sie mit Salzen oder durch Einstellung des pH-Wertes auf den isoelektrischen Punkt (pH 4,5 bis 7,0, vorzugsweise 5,5 bis 6,5) behandelt. Durch Neutralisation, also Veränderung des pH's erhält man erfahrungsgemäß etwas härtere Fasern als durch Entquellung mit Hilfe von Salzen. Geeignete Salze für die Entquellung sind Natriumchlorid oder Natriumsulfat, wobei Natriumchlorid wiederum bevorzugt wird. Eine Entquellung mit anderen Salzen ist grundsätzlich möglich, jedoch ist darauf zu achten, daß diese Salze so leicht entfernbar sind, und entfernt werden können, daß sie bei der späteren Verwendung nicht mehr vorhanden sind, oder nicht stören. Deshalb wird Kochsalz und zwar in einer Konzentration von 5 bis 50 Gew. %, vorzugsweise 7 bis 15 Gew. %, zum Entquellen verwendet.

Die bei der Entquellung freigegebene Flüssigkeit wird abgetrennt, z. B. durch Zentrifugieren oder in anderer geeigneter Weise. Während des Entquellens der Fasern kann man vorteilhaft eine Imprägnierung mit Arzneimitteln vornehmen, sofern dies erwünscht ist.

Die entquollenen Fasern werden anschließend vernetzt. Durch die Gerbung wird die Wasserbeständigkeit der Fasern erheblich verbessert und die Antigenizität und Allergieaktivität des Material weitgehend eliminiert. Auch die Weiterverarbeitung zu Textilien wird erleichtert.

Für die Vernetzung von Kollagenfasern sind zahlreiche Materialien bekannt, die bei der Behandlung von Kollagenfasern für medizinische Zwecke und auch bei der Lederherstellung schon verwendet wurden.

Untersuchungen haben gezeigt, daß die Vernetzung mit Aldehyden, wie dies z. B. aus der DE-B-27 30 623 bekannt ist, z. B. Formaldehyd, nicht zu physiologisch einwandfreien Kollagenfasern führt. Dagegen erhält man bei der Verwendung von Hexamethylendiisocyanat als Vernetzungs- bzw. Chromierungsmittel Fasern, die textiltechnisch gut verarbeitbar sind, und ein physiologisch absolut einwandfreies Kollagenmaterial ergeben.

Die Vernetzung kann in der wässrigen Salzlösung, wie sie nach dem Entquellen anfällt, durchgeführt werden. In diesem Fall verzichtet man auf die Abtrennung der Salzlösung nach dem Entquellen zu diesem Zeitpunkt. Wurde die wässrige Lösung bereits nach dem Entquellen abgetrennt, so wird eine entsprechende Menge Flüssigkeit wieder zugegeben.

Die Vernetzung wird mit Hexamethylendiisocyanat in Gegenwart von nichtionischen oder kationischen Emulgatoren vorgenommen. Hierzu verwendet man einen stöchiometrischen Überschuß an Hexamethylendiisocyanat. In der Praxis sind dies etwa 10 bis 20 Gew. % Hexamethylendiisocyanat, bezogen auf das Trockengewicht des Kollagenmaterials. Die Reaktion, bei welcher eine Vernetzung erfolgt, verläuft verhältnismäßig langsam und man benötigt 10 bis 40, vorzugsweise 15 bis 30 Studen. Die bei der Vernetzungsreaktion entstehenden Nebenprodukte, wie Hexamethylendiamin, sowie die verwendeten Emulgatoren, und das gegebenenfalls aus der Entquellung noch vorhandene Salz werden anschließend beim Trocknen der vernetzten Kollagenfasern entfernt.

Die Trocknungs- bzw. Entwässerungsstufe erfolgt in für Kollagenfasern bekannter Weise. Das Trocknen kann mit Hilfe von organischen Lösungsmitteln, die mit Wasser mischbar sind, und niedrige Siedepunkte haben, durchgeführt werden, jedoch hat sich Aceton als besonders geeignetes Lösungsmittel herausgestellt.

Das nunmehr getrocknete, gegerbte, vernetzte, längsorientierte Fasermaterial kann jetzt auf speziellen Textilmaschinen verarbeitet werden. Dabei ist es für den Fachmann selbstverständlich, daß man bei der Verwendung von Textilmaschinen die besonderen Eigenschaften der Kollagenfasern berücksichtigt. Dies ist dem Fachmann geläufig, weil er z. B. weiß, daß Leinen in anderer Weise als Baumwolle auf Textilmaschinen behandelt werden muß. So kann man die Kollagenfasern auf modifizierten Krempeln zu Faservliesen von beliebigem Gewicht verarbeiten und anschließend mit Prägewalzen zu festen flexiblen Flächengebilden verfestigen.

Weiterhin kann das verfestigte Faservlies zu einem dem Streichgarn sehr ähnlichen Fasergarn verarbeitet werden, wobei die vom Garn abstehenden Fasern durch eine Nachbehandlung in einem Nitschelhosenpaar angelegt werden, und die so hergestellten Garne auf herkömmlichen Textilmaschinen zu Geweben und Gewirken verarbeitet werden.

Die erfindungsgemäß hergestellten Fasern sind wesentlich länger als Kollagenfasern, die in bekannter Weise hergestellt werden. Die Faserlänge liegt zwischen 40 mm bis 100 mm und die Faserdicke beträgt ca. 30 bis 80 $\mu$m.

Die vorstehenden Ausführungen lassen erkennen, daß beim erfindungsgemäßen Verfahren zur Herstellung von Kollagenfasern für chirurgische Zwecke eine Reihe von Stufen durchlaufen wird, die zum Teil als Einzelstufen schon bei der Herstellung von Kollagenfasern angewendet worden sind. Es kommt beim erfindungsgemäßen Verfahren aber auf die genaue Einhaltung der Stufen und der in diesen Stufen genannten Bedingungen sowie auf die genaue Reihenfolge dieser Stufen an.

Erfindungsgemäß erhält man ein noppenfreies und allergenfreies Kollagenfasermaterial, aus dem sich ein chirurgisches Material für zahlreiche Anwendungsmöglichkeiten mit ausgezeichneten Eigenschaften ergibt.

Beispiel 1

Achillessehnen von Rindern werden unmittelbar nach dem Schlachten mechanisch von Fettresten gereinigt und mit Wasser gewaschen. Aus einem Rind kann man etwa 250 g Achillessehnen (Trockengehalt etwa 30 %) gewinnen.

Die gewaschenen Achillessehnen werden acht Tage bei 20 °C in einer Lösung von 20 % Natriumsulfat, 2 % Natriumhydroxid und 150 % Wasser, bezogen auf das Naßgewicht der Sehnen, behandelt. Am zweiten, vierten und sechsten Tag werden die Sehnen zwischen Riffelwalzen gepreßt. Anschließend wäscht man mit fließendem Wasser. Der Amidstickstoffgehalt beträgt nach dem Waschen 0,23 $\mu$Mol/g.

Anschließend behandelt man die Sehnen in einem Gerbfaß mit Salzsäure. Die Gerbflotte besteht aus 100 % Wasser und 10 % einer 3 %-igen Salzsäure, bezogen auf das Naßgewicht der Sehnen. Die Salzsäurebehandlung dauert etwa 4 Stunden. Anschliessend wäscht man das Material unter fließendem Wasser bis zu einem pH-Wert der Sehnen (gleichmäßig verteilt in der ganzen Dicke) zwischen 2,7 bis 3,3. Das anfallende Material hat ein Trockengewicht von etwa 14 %, bezogen auf die nassen Sehnen.

In einer Hammerpresse werden die stark aufgequollenen Sehnen so stark behandelt, daß überschüssiges Wasser, lösliche und denaturierte Eiweißstoffe beseitigt werden. Die Sehnen liegen nunmehr in Form eines fasrigen, zweidimensionalen Netzwerkes vor. Diese fasrige Masse wird in einer Hechelmaschine bearbeitet, wobei man die Behandlung dreimal wiederholt und die Kämmintensität kontinuierlich erhöht.

Zu den gehechelten Fasern gibt man 10 Gew.-Teile Kochsalz und 100 Gew.-Teile Wasser, bezogen auf das Naßgewicht der Fasern, und läßt das Material über Nacht zum Entquellen stehen. Die überstehende Flüssigkeit wird am Folgetag abzentrifugiert.

Das entquollene Material wird in einen Behälter zusammen mit einer Kochsalzlösung (100 %, bezogen auf das Naßgewicht der Fasern) gegeben. Die Konzentration des Salzes ist 5 %. In den Behälter gibt man 1 Gew. %, bezogen auf das Naßgewicht der Fasern, eines nichtionischen Emulgators. Der pH-Wert wird mit Natriumhydroxid auf 6,0 eingestellt. Nach 30-minütigem Bewegen in dem Faß werden 5 Gew. % Hexamethylendiisocyanat, bezogen auf das Naßgewicht der Fasern, zugegeben, und die Masse wird 30 Stunden unter ständigem Bewegen im Faß behandelt. Hexamethylendiisocyanatdämpfe werden dabei ständig abgesaugt. Nach der Behandlung wird die Flüssigkeit abzentrifugiert.

Anschließend wird das vernetzte Material mit Aceton entwässert, wobei viermal je 100 Gew. % Aceton, bezogen auf das Naßgewicht der Fasern (nach dem Zentrifugieren), verwendet wird. Die so erhaltenen Fasern werden an der Luft getrocknet und können, z. B. für Wundabdeckungen in folgender Weise weiterverarbeitet werden:

Die Fasern werden auf einem Krempel aufgelöst. Die Krempel besteht aus einer Vorkrempel, die zwei bis drei Arbeitsstellen hat und mit einer Sägezahngarnitur bezogen ist. Daran schließt sich ein Hauptkrempel mit etwa 5 Arbeitsstellen an, die mit einer flexiblen Garnitur besetzt ist.

Durch eine spezielle Einstellung der Kratzen zueinander kommt eine gute Auslösung der Fasern zustande. Am Ende der Maschine liegt ein Flor vor. Durch Doublieren dieses Flors werden Vliese mit einem beliebigen Gewicht hergestellt. Eine Verbesserung des Flors und damit der Vliese in bezug auf Gleichmäßigkeit kann durch eine weitere Krempelung mit feineren Garnituren und engeren Einstellungen erreicht werden. Der zu einem Vlies doublierte Flor läßt sich durch Kalandrieren mit einer Frägewalze zu einem festen Flächengebilde verarbeiten. Die erhaltenen Vliese sind für Wundabdeckung geeignet und haben in nichtgegerbtem (unvernetztem) Zustand eine ausgezeichnete hämostatische Wirkung.

Zur Herstellung von Garn aus den erfindungsgemäß aufgeschlossenen Kollagenfasern wird das wie vorher angegeben auf einer Krempel aufgelöste und zu einem Flor verarbeitete Fasergut in einem Florteiler in Bändchen geteilt. Die erhaltenen Faserbändchen werden durch Nitscheln gerundet und zu einem Vorgarn geformt, das allerdings noch keine Festigkeit hat.

Das Vorgarn wird auf einer Streichgarnringspinnmaschine verstreckt und gedreht. Die Anzahl der Garndrehungen hängtvorwiegend von einer erstrebten Garnfestigkeit und von der Faserlänge ab. Dabei kann man die Spindelgröße der Ringdurchmesser, die Ringläufer und die Laufgeschwindigkeit den jeweiligen Erfordernissen anpassen.

Das fertige Garn hat meistens viele aus dem Fadenkern herausstehende Faserenden, die ein Verweben oder Verstricken noch sehr erschweren. Deshalb wird dieses Garn durch angefeuchtete Nitschelhosen laufengelassen, wobei die abstehenden Fasern an den Fadenkern angelegt werden.

Aus diesen Fäden kann man nun in an sich üblicher Weise Gewebe oder Gewirke herstellen.

**Patentansprüche**

1. Verfahren zur Herstellung von Kollagenmaterial für chirurgische Zwecke, bei dem man Achillessehnen oder Häute alkalisch aufschließt und nach einer Säurebehandlung mechanisch behandelt, entquellt, vernetzt und trocknet, dadurch gekennzeichnet, daß man die Achillessehnen oder Häute alkalisch bis zu einem Amidstickstoffgehalt von 0,15 bis 0,30 µMol/g aufschließt und nach einer Salzsäurebehandlung und Waschen auf einen pH-Wert von 2 bis 4 die angequollene Kollagenmasse unter Druck unter Ausbildung eines zweidimensionalen Netzwerkes in der Dicke reduziert, dann durch Hecheln einzelne längsorientierte Fasern freilegt und dann nach in an sich bekannter Weise erfolgter Entquellung die Fasern mit Hexamethylendiisocyanat in Gegenwart eines nichtionischen oder kationischen Emulgators vernetzt und trocknet und auf Textilmaschinen weiter verarbeitet.

2. Verfahren gemäß Anspruch 1, dadurch gekennzeichnet, daß man die Dickenreduzierung der angequollenen Kollagenmasse mit einer Hammerpresse oder einem Kalander oder mit Riffelwalzen durchführt.

3. Verfahren nach Ansprüchen 1 oder 2, dadurch gekennzeichnet, daß die Entquellung der Fasern mit Kochsalzlösung einer Konzentration von 5 bis 50 Gew.%, vorzugsweise 7 bis 15 Gew.%, vorgenommen wird.

4. Verfahren nach Anspruch 1, dadurch gekennzeichnet, daß man während der Entquellung der Fasern gleichzeitig eine Imprägnierung mit Medikamenten vornimmt.

5. Verfahren nach Ansprüchen 1 bis 4, dadurch gekennzeichnet, daß Kollagenfasern auf modifizierten Krempeln zu Faservliesen von beliebigem Gewicht verarbeitet und anschliessend durch Pressen mit Prägewalzen zu festen textilen Flächengebilden verfestigt werden.

6. Verfahren nach Anspruch 5, dadurch gekennzeichnet, daß das verfestigte Faservlies zu einem dem Streichgarn sehr ähnlichen Fasergarn verarbeitet wird, wobei die vom Garn abstehenden Fasern durch eine Nachbehandlung in einem Nitschelhosenpaar angelegt werden, und die so hergestellten Garne auf herkömmlichen Textilmaschinen zu Geweben und Gewirken verarbeitet werden.

**Claims**

1. A process for the manufacture of collagen fibers for surgical purposes, in which Achilles tendones or hides are decomposed in an alkaline medium and are mechanically treated after treatment with an acid, deswelled, cross-linked and dried, characterized in that said Achilles tendons or hides are decomposed in an alkaline medium to an amino-nitrogen content of from 0.15 to 0.30 µmoles/g and after a hydrochloric acid treatment and washing to a pH value of from 2 to 4, the swollen collagen substance is reduced in thickness under pressure forming a two-dimensional network, then the individual longitudinal fibers are exposed by hackling, and then, after deswelling in a manner known per se the fibers are cross-linked with hexamethylene diisocyanate in the presence of a nonionic or cationic emulsifier and are dehydrated and further processed on textile machines.

2. Process according to claim 1, characterized in that the thickness reduction of the swollen collagen substance is carried out by using a hammer press or a calendering machine or by fluted rollers.

3. Process according to claims 1 or 2, characterized in that the deswelling of the fibers is carried out by the use of a sodium chloride solution having a concentration of from 5 to 50 % by weight, preferably 7 to 15 % by weight.

4. Process according to claim 1, characterized in that an impregnation with medicaments at the same time as the deswelling of said fibers is carried out.

5. Process according to claims 1 to 4, characterized in that the collagen fibers are processed on modified carding machines to fiber fleeces of any desired weight and then by pressing with embossing rollers are solidified into firm textile flat shaped articles.

6. Process according to claim 5, characterized in that the solidified fiber fleece is processed to fiber yarn very similar to carded yarn, whereby said fibers protruding from the yarn are pieced by a subsequent treatment into a pair of rubbing leathers, and the yarns thus prepared are processed on conventional textile machines into woven fabrics and knitted goods.

**Revendications**

1. Procédé de préparation de matière collagène à usage chirurgical, selon lequel on désagrège par voie alcaline des talons d'Achille ou des peaux et, après un traitement acide, on les traite mécaniquement, on les fait dégonfler, on les fait réticuler et on les fait sécher, caractérisé en ce qu'on fait subir aux talons d'Achille ou aux peaux une désagrégation alcaline jusqu'à obtention d'une teneur en azote amidé de 0,15 à 0,30 µmole/g, et, après traitement à l'acide chlorhydrique et lavage à une valeur du pH de 2 à 4, on effectue une réduction sous pression de l'épaisseur de la masse de collagène gonflé avec formation d'une structure réticulée bidimensionnelle, puis l'on en dégage par peignage des fibres individuelles orientées longitudinalement, et après le dégonflage effectué de façon connue, on provoque la réticulation des fibres avec de l'hexaméthylène-diisocyanate en présence d'un agent émulsifiant non ionique ou cationique et on les fait sécher, et on les met en œuvre ensuite sur des machines textiles.

2. Procédé selon la revendication 1 caractérisé en ce qu'on effectue la réduction d'épaisseur de la masse de collagène gonflé au moyen d'une presse à marteaux ou d'une calandre, ou au moyen de cylindres cannelés.

3. Procédé selon les revendications 1 ou 2 caractérisé en ce qu'on effectue le dégonflage des fibres à l'aide d'une solution de sel de cuisine à une concentration de 5 à 50 % en poids, de préférence de 7 à 15 % en poids.

4. Procédé selon la revendication 1 caractérisé en ce qu'on effectue, en même temps que le dégonflage des fibres, une imprégnation par des médicaments.

5. Procédé selon les revendications 1 à 4 caractérisé en ce qu'on convertit des fibres de collagène, en voiles cardés d'un poids quelconque, à l'aide de machines à carder modifiées, et on les consolide ensuite à la presse à l'aide de rouleaux de gaufrage, en produits textiles plats compacts.

6. Procédé selon la revendication 5 caractérisé en ce qu'on convertit le voile cardé compact en un filé de fibres très semblable à un fil cardé, après quoi les fibres saillantes hors du filé sont redisposées par un traitement subséquent à l'aide d'une paire de manchons frotteurs, et on transforme les fils ainsi fabriqués en tissus et en tricots sur des machines textiles courantes.